# EUROPÄISCHE PATENTANMELDUNG

(11) **EP 2 298 373 A1**
(43) Veröffentlichungstag der Anmeldung: **23.03.2011**
(21) Anmeldenummer: 09075441.7
(22) Anmeldetag: 22.09.2009
(51) Int. Cl.: A61M 1/10, A61M 1/12, F04D 29/24

(54) **Fluidpumpe mit wenigstens einem Schaufelblatt und einer Stützeinrichtung**

(71) Anmelder: ECP Entwicklungsgesellschaft mbH, 12247 Berlin (DE)
(72) Erfinder: Schumacher, Jörg, 14513 Teltow (DE); Röhn, Daniel, 12557 Berlin (DE)
(74) Vertreter: Pfenning, Meinig & Partner GbR

(57) **Zusammenfassung**

Die Erfindung bezieht sich auf eine Fluidpumpe mit wenigstens einem Schaufelblatt (1,1',1"), das um eine Rotationsachse (3) drehbar ist und im Betrieb ein Fluid fördert und mit einer Stützeinrichtung (4,6,7,8,9,10,12,12',13,13',14,14',15,17), die das wenigstens eine Schaufelblatt (1,1',1") in wenigstens einem Stützbereich stützt, wobei die Stützeinrichtung zwischen einem ersten Zustand, in dem der Rotor radial komprimiert ist, und einem zweiten Zustand, in dem der Rotor radial expandiert ist, veränderbar ist und wobei sich wenigstens ein Schaufelblatt im radial expandierten Zustand des Rotors wenigstens teilweise in Bezug auf die Rotationsachse (3) von dem Stützbereich / den Stützbereichen aus radial nach innen erstreckt.

## Beschreibung

Die Erfindung liegt auf dem Gebiet des Maschinenbaus, insbesondere der Mikromechanik, und befasst sich mit Fluidpumpen, die mit rotierenden Schaufelblättern arbeiten und besonders zum Einsatz in schwierig zugänglichen Bereichen eingerichtet sind.

Derartige Pumpen können beispielsweise im medizinischen Bereich eingesetzt werden und zu diesem Zweck auch besonders kleine Bauformen aufweisen.

Eine besondere Anwendung von Mikropumpen ist beispielsweise die Unterstützung der Pumpkraft des menschlichen Herzens. Pumpen, die in diesem Bereich eingesetzt werden, werden üblicherweise durch Blutgefäße in den Körper eingeführt und gegebenenfalls in einer Herzkammer betrieben.

Es ist eine Vielzahl solcher Pumpen bereits bekannt geworden, die verschiedenste Bauformen aufweisen. Aus der WO 98/53864 ist, ebenso wie aus der EP 1 738 783 A1, eine Axialpumpe bekannt geworden, die jeweils einen mit Schaufelblättern versehenen Läufer in Form eines starren Rohres aufweist, der außen in einem Stator gelagert ist. Der Antrieb kann direkt in den Stator und den Rotor als elektromagnetischer Antrieb integriert sein.

Derartige Pumpen haben den Nachteil, dass sie einen im Verhältnis zur Pumpleistung großen Durchmesser aufweisen und kaum durch ein Blutgefäß einzuführen sind.

Im Gegensatz dazu ist aus der WO 03/103745 A2 ein Rotor bekannt, der in einem komprimierten Zustand einen geringeren Durchmesser aufweist als in einem expandierten Zustand und der ein entfaltbares Rotorblatt aufweist, das sich im Betrieb durch den Fluidgegendruck des Blutes entfaltet.

Andere bekannt gewordene Rotoren weisen ebenfalls Schaufelblätter auf, die zum Betrieb entfaltbar sind, beispielsweise durch Gelenke oder durch elastische Verformbarkeit der Schaufelblätter.

Ein besonderes Problem ist hierbei, dass die Schaufelblätter üblicherweise an einer zentrischen Nabe befestigt und von dieser aus rotatorisch antreibbar und auch beweglich schwenkbar sind, dass die Schaufelblätter somit flexibel sein, jedoch andererseits eine gewisse Steifigkeit bzw. eine Begrenzung ihrer Beweglichkeit aufweisen müssen, um den notwendigen Druck auf das Fluid zur Förderung auszuüben.

Diese Aufgabe wurde im Stand der Technik bisher noch nicht optimal gelöst. Der vorliegenden Erfindung liegt daher die Aufgabe zugrunde, eine Pumpe der beschriebenen Art weiterzuentwickeln, um trotz eines im komprimierten Zustand geringen Pumpendurchmessers im Betrieb eine gute Pumpleistung zu erreichen. Die Konstruktion soll
dabei möglichst unaufwendig und kostengünstig sein.

Die Aufgabe wird gemäß der Erfindung mit den Merkmalen des Patentanspruchs 1 gelöst.

Dabei ist wenigstens ein Schaufelblatt vorgesehen, das um eine Rotationsachse drehbar ist, um Fluid zu fördern, sowie eine Stützeinrichtung, die das wenigstens eine Schaufelblatt in einem Stützbereich stützt. Die Stützeinrichtung ist zudem zwischen einem komprimierten Rotorzustand und einem expandierten Rotorzustand veränderbar, und wenigstens ein Teil wenigstens eines Schaufelblattes erstreckt sich im expandierten Rotorzustand wenigstens teilweise vom Stützbereich aus gesehen radial nach innen auf die Rotorachse hin. Dadurch, dass der Stützbereich nicht am radial inneren Ende des Schaufelblattes liegt, sondern zum Schaufelradäußeren radial betrachtet versetzt ist, wird das Schaufelblatt/werden die Schaufelblätter in einem Bereich gestützt, in dem die Relativgeschwindigkeit zum Fluid größer ist als im Bereich der Rotationsachse und gegebenenfalls die mechanische Belastung des Schaufelblattes entsprechend stärker ist. Der Stützbereich und die Stützeinrichtung kann der einzige Bereich sein, in dem das Schaufelblatt /die Schaufelblätter gelagert oder mit einem anderen Bauteil verbunden oder zumindest der einzige Bereich, indem es/sie mittels der Stützeinrichtung mit anderen Bauteilen kraftübertragend verbunden ist/sind. Dabei kann der Fluid fördernde Bereich des Schaufelblattes / der Schaufelblätter ganz oder nur teilweise radial innerhalb des Stützbereiches liegen. Jedenfalls wird hier an die Stützeinrichtung und ihre Verbindung zu dem jeweiligen Schaufelblatt eine geringere mechanische Anforderung gestellt, als wenn die Stützeinrichtung die Schaufelblätter im Bereich der Rotationsachse stützen würde. Die Schaufelblätter können zudem schwächer ausgeführt werden, da sie in einem Bereich höherer Belastung gestützt werden und der mittlere Abstand der Fluid fördernden Bereiche des Schaufelblattes / der Schaufelblätter, in radialer Richtung betrachtet, vom Stützbereich geringer ist, als wenn dieses/diese im Bereich einer Nabe auf der Rotationsachse gestützt wäre(n).

Es kann auch vorgesehen sein, dass sich der überwiegende Teil des Fluid fördernden Bereiches des Schaufelblattes / der Schaufelblätter radial innerhalb des Stützbereiches / der Stützbereiche erstreckt. Damit ist der Stützbereich den sich am schnellsten bewegenden Teilen des Schaufelblattes / der Schaufelblätter am nächsten und kann diese effizient stützen, beispielsweise ein Drehmoment auf diese übertragen.

Vorteilhaft kann auch vorgesehen sein, dass der Stützbereich radial außen am Umfang des Fluid führenden Bereiches des Schaufelblattes / der Schaufelblätter angeordnet ist. Dabei kann der Stützbereich die Schaufelblätter vollständig radial außen umgeben.

Grundsätzlich kann das Schaufelblatt mit der Stützeinrichtung fest verbunden sein, so dass diese mit dem Schaufelblatt rotiert.

Dies ist eine Bauform, die besonders einfach herzustellen und mechanisch stabil ist. Die Stützeinrichtung kann beispielsweise aus demselben Material bestehen wie das Schaufelblatt und einstückig mit diesem hergestellt sein.

Es kann jedoch auch vorgesehen sein, dass die Stützeinrichtung aus einem anderen Material hergestellt ist als das Schaufelblatt, beispielsweise aus einer superelastischen Verbindung bzw. einem Formgedächtnismaterial, insbesondere Nitinol, so dass die Stützeinrichtung vor dem Übergang in den Betriebszustand aktiv in eine Betriebsgestalt übergehen kann, um damit den Rotor mit den Schaufelblättern aufzurichten, damit an die Schaufelblätter keine weiteren Anforderungen in Bezug auf eine selbsttätige Verformung gestellt werden. Diese können dann als dünne, nicht selbsttragende biegeschlaffe Folien hergestellt sein.

Das wenigstens eine Schaufelblatt kann jedoch auch beweglich gegenüber der Stützeinrichtung geführt und gelagert sein. Dann kann die Stützeinrichtung als Stator gegenüber den Schaufelblättern stillstehen. Es ist dann eine Führung beispielsweise in Form einer mechanischen oder magnetischen Lagerung der Schaufelblätter gegenüber der Stützeinrichtung notwendig.

Die Stützeinrichtung kann beispielsweise durch wenigstens einen zur Rotationsachse konzentrisch positionierten und gegebenenfalls gelagerten Ring gebildet sein. Dieser Ring kann eine axiale Länge aufweisen, die geringer als die axiale Länge der Schaufelblätter ist.

Es können auch zwei oder mehr solche Ringe axial voneinander beabstandet jeweils mit dem Schaufelblatt / den Schaufelblättern verbunden sein. Die Ringe können beispielsweise in Umfangsrichtung mäanderförmig ausgestaltet sein, um eine entsprechende Verformbarkeit beispielsweise infolge von Superelastizität bzw. Formgedächtniseigenschaften besonders einfach realisieren zu können. Mehrere Ringe sind vorzugsweise koaxial zueinander angeordnet.

Die Stützeinrichtung kann jedoch auch durch einen das Schaufelblatt / die Schaufelblätter umgebenden flexiblen Schlauch gebildet sein. Ein solcher Schlauch kann selbst aus einem Formgedächtnismaterial bestehen, beispielsweise auch aus einem Maschendrahtgeflecht aus Nitinoldraht, oder er kann aus einem für das Fluid undurchlässigen flexiblen organischen Material bestehen und Stützelemente wie beispielsweise Stützringe aufweisen. Der Schlauch kann im Pumpbetrieb durch Überdruck infolge des aufgebauten Fluiddrucks aufblasbar sein.

Der Schlauch kann punktuell oder streckenweise mit den äußeren Enden des Schaufelblattes / der Schaufelblätter verbunden sein.

Gemäß der vorliegenden Erfindung benötigt der Rotor im Bereich der Schaufelblätter keine Nabe, so dass das Schaufelblatt / die Schaufelblätter radial mit allen seinen / ihren Teilen von der Rotationsachse beabstandet sein kann / können. In diesem Fall steht der ansonsten durch eine Nabe beanspruchte Querschnitt des Rotors zusätzlich zur Förderung von Fluid zur Verfügung.

Wenn sich die Stützeinrichtung mit dem Schaufelblatt / den Schaufelblättern dreht, so kann sie kann in wenigstens einem Rotationslager gelagert sein, das axial außerhalb des Bereiches angeordnet ist, über den sich das Schaufelblatt / die Schaufelblätter erstreckt / erstrecken.

Diese Konstruktion ermöglicht eine einfache Lagerung in einem handelsüblichen Rotationslager, beispielsweise einem Wälzlager oder einem magnetischen Lager. Eine solche Lagerung ist weniger aufwendig und reibungsärmer als eine Lagerung am
Umfang der Stützeinrichtung im Bereich der Schaufelblätter.

Es kann aber auch eine beispielsweise hydrodynamische Lagerung am Umfang der Stützeinrichtung vorgesehen sein, wenn ein Rotor der beschriebenen Art in einem Gehäuse läuft, und zwischen dem Gehäuse und der Stützeinrichtung ein Spalt vorgesehen ist, in dem sich ein Fluid befindet. In einer besonders einfachen Ausführung könnte dieses Fluid mit dem geförderten Fluid identisch sein.

Im Folgenden wird die Erfindung anhand eines Ausführungsbeispiels in einer Zeichnung gezeigt und anschließend beschrieben. Dabei zeigt
- Fig. 1: eine dreidimensionale, teilweise durch- brochene Ansicht eines Rotors einer Fluidpumpe mit einer Stützeinrichtung und einem Schaufelblatt,
- Fig. 2: eine axiale Draufsicht auf den Gegen- stand aus Fig. 1,
- Fig. 3: eine Seitenansicht,
- Fig. 4: eine teilweise durchbrochene Seiten- ansicht des Gegenstandes aus Fig. 1,
- Fign. 5 & 6: jeweils Längsschnitte des Gegenstandes aus Fig. 1,
- Fig. 7: einen Rotor mit einer aus zwei Ringen bestehenden Stützeinrichtung und einem Schaufelblatt in dreidimensionaler An- sicht,
- Fig. 8: den Gegenstand aus Fig. 7 in einem Längsschnitt,
- Fig. 9: den Gegenstand aus Fig. 7 in einer ersten Seitenansicht,
- Fig. 10: den Gegenstand aus Fig. 7 in einer zweiten Seitenansicht,
- Fig. 11: den Gegenstand aus Fig. 7 in einer axialen Draufsicht,
- Fig. 12: den Gegenstand aus Fig. 7 in einem gegenüber dem Längsschnitt aus Fig. 8 winkelmäßig versetzten Längsschnitt,
- Fig. 13: einen Rotor mit einer aus drei Ringen bestehenden Stützeinrichtung,
- Fig. 14: eine Seitenansicht des Rotors aus Fig. 13,
- Fig. 15: einen Rotor mit einer vier Ringe auf- weisenden Stützeinrichtung in drei- dimensionaler Ansicht,
- Fig. 16: einen Rotor, bei dem die Stützeinrich- tung aus mindestens einem Schlauchstück besteht,
- Fig. 17: einen Längsschnitt des Gegenstandes aus Fig. 16,
- Fig. 18: einen Rotor, bei dem das Schaufelblatt / die Schaufelblätter zur Gänze von einer schlauchartigen Stützeinrichtung umgeben sind, wobei Verstärkungsringe den Schlauch verstärken,
- Fig. 19: den Rotor aus Fig. 18 in einem Längs- schnitt,
- Fig. 20: den Rotor aus Fig. 18 in einer Seiten- ansicht,
- Fig. 21: den Rotor aus Fig. 18 in einer drei- dimensionalen Ansicht, wobei die Stütz- ringe in Umfangsrichtung mäanderförmig gestaltet sind,
- Fig. 22: einen Rotor mit einer schlauchartigen Stützeinrichtung, die durch ein Maschendrahtgeflecht verstärkt ist, in dreidimensionaler Ansicht,
- Fig. 23: einen Rotor ähnlich dem aus Fig. 22, wobei zwei Rotorblätter ohne eine Nabe derart angeordnet sind, dass sie nicht bis zur Rotationsachse reichen und beim Komprimieren des Rotors aneinander vor- beigleiten können,
- Fig. 24: die Anordnung aus Fig. 23 in einem Längsschnitt,
- Fig. 25: eine Rotoranordnung mit einem Schaufel- blatt, das von einer schlauchartigen Stützeinrichtung umgeben ist, wobei die Stützeinrichtung mittels gabelförmiger Streben beiderseits an je einen Wellen- zapfen angeschlossen ist,
- Fig. 26: eine Seitenansicht des Gegenstandes aus Fig. 25,
- Fig. 27: eine teilweise durchbrochene Ansicht des Gegenstandes aus Fig. 26,
- Fig. 28: eine dreidimensionale Außenansicht des Gegenstandes aus den Fign. 25-27,
- Fig. 29: eine teilweise durchbrochene dreidimen- sionale Ansicht des Gegenstandes aus Fig. 28,
- Fig. 30: einen Rotor mit einer schlauchförmigen Stützeinrichtung, die über eine Strebe an einen Wellenzapfen angeschlossen ist, wobei ein einziges Schaufelblatt vorgesehen ist,
- Fig. 31: die Anordnung aus Fig. 30 in teilweise durchbrochener Ansicht,
- Fig. 32: eine Ausgestaltung ähnlich der Kon- struktion aus Fig. 30, wobei das Schau- felblatt direkt und nicht über die Stützeinrichtung an den Wellenzapfen angekoppelt ist,
- Fig. 33: die Konstruktion aus Fig. 32 in einem Längsschnitt.

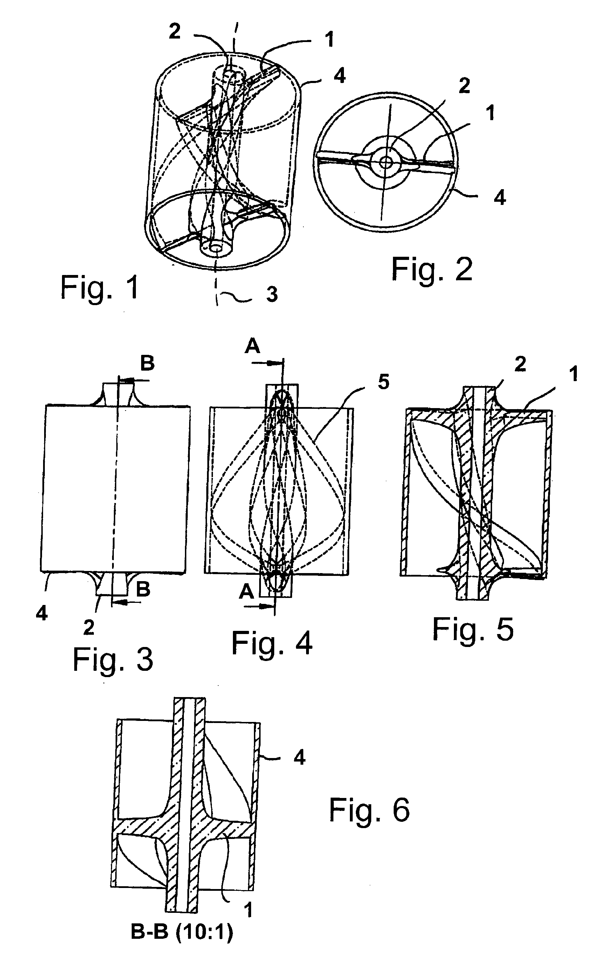

Fig. 1 zeigt in dreidimensionaler Ansicht einen Rotor einer Fluidpumpe, insbesondere einer Mikropumpe zur axialen Förderung von Blut, wie sie üblicherweise in der Medizin zur Unterstützung des menschlichen Herzens eingesetzt wird. Eine derartige Pumpe ist beispielsweise am Ende eines Hohlkatheters montiert und führt, wenn sie durch ein Blutgefäß in ein Herzventrikel eingeführt ist, Blut unter Druck aus einer Herzkammer in ein Blutgefäß. Dazu rotiert ein Rotor mit einigen tausend Umdrehungen pro Minute, um die notwendige Förderleistung zu erreichen. Das Schaufelblatt 1 ist wendelförmig ausgebildet, mit einer Nabe 2 im Bereich der Rotationsachse 3 verbunden und außen durch eine Stützeinrichtung 4 in Form einer schlauchartigen Hülle gestützt, mit der das Schaufelblatt 1 an seinem äußeren Rand verbunden ist.

Die Nabe 2 ist üblicherweise mit einer antreibbaren Welle verbunden, die durch den Hohlkatheter und ein Blutgefäß zu einem Motorantrieb verläuft, der üblicherweise außerhalb des Körpers angeordnet sein kann. Zwischen dem Motorantrieb und dem Hohlkatheter ist eine Schleuse vorgesehen.

Fig. 2 zeigt eine Draufsicht, bei der der obere Rand des Schaufelblatts 1 sowie die schlauchartige Hülle 4 gut zu erkennen ist.

Das Schaufelblatt 1 kann auch als zwei Teilschaufelblätter aufgefasst werden, die sich radial jeweils von der Nabe 2 zur schlauchförmigen Stützeinrichtung 4 hin erstrecken und axial wendelförmig verlaufen.

Fig. 3 zeigt den Rotor aus der Fig. 1 in einer Seitenansicht, wobei die geschlossene rohr- oder schlauchartige Hülle 4 zu erkennen ist sowie die über diese hinausragenden Enden der Nabe 2.

Fig. 4 zeigt eine durchbrochene Darstellung des Rotors aus Fig. 3, wobei die Randbereiche des Schaufelblattes 1 dort, wo es mit der Hülle/Stützeinrichtung 4 verbunden ist, gestrichelt dargestellt sind.

Fig. 5 zeigt einen Längsschnitt durch den Rotor aus Fig. 1, wobei am oberen Ende des Rotors das Schaufelblatt 1 die Zeichenebene schneidet.

Das Schaufelblatt kann, wie in Fig. 5 und auch in der nachfolgenden Fig. 6 dargestellt, einstückig mit der als kollabierbarer Schlauch oder als kollabierbares Rohr ausgebildeten Stützeinrichtung 4 hergestellt sein. Diese kann beispielsweise als flexibler Schlauch aus einem Kunststoff bestehen und durch die Pumpwirkung infolge des in seinem Inneren aufgebauten Überdrucks expandiert und formstabil gehalten werden. Die Formstabilität kann aber auch durch die elastischen Rückstellkräfte des Materials hergestellt werden. Gleichzeitig werden mit der Expansionsbewegung die Schaufelblätter expandiert und in die betriebsbereite Form gebracht. Das Schaufelblatt 1 beispielsweise wird zwischen der Stützeinrichtung 4 und der Nabe 2 im expandierten Zustand durch Zug gespannt und damit gut stabilisiert.

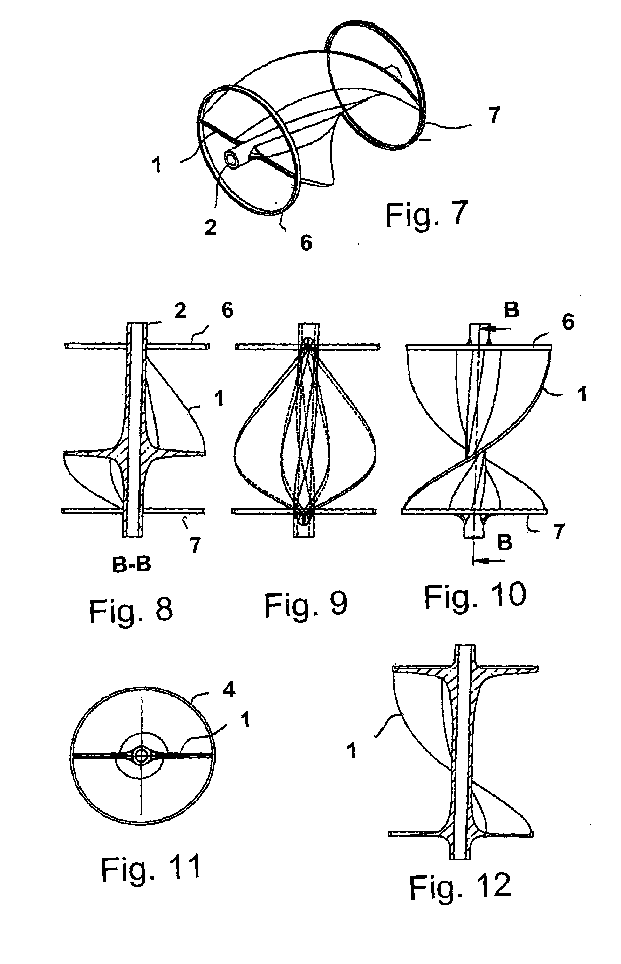

Aus der Fig. 7 ist eine Ausführungsform mit zwei Ringen 6, 7 ersichtlich, die gemeinsam die Stützeinrichtung bilden und das Schaufelblatt 1 stützen. Die Stützbereiche des Schaufelblattes 1 liegen dabei radial dessen äußerstem Rand.

Die Ringe 6, 7 können aus einer Formgedächtnislegierung, beispielsweise Nitinol, bestehen und nach dem Einführen in einen Körper gezielt expandiert werden, um die dargestellte Kreisform anzunehmen. Gleichzeitig ziehen sie die Stützbereiche des Schaufelblattes 1 radial nach außen und spannen dieses.

Fig. 8 zeigt einen Längsschnitt durch den Rotor gemäß Fig. 7 und Fig. 9 den Verlauf des Randes des Schaufelblattes in einer Seitenansicht.

Fig. 10 zeigt eine dreidimensionale Seitenansicht, die die Wendelstruktur des Schaufelblattes 1 deutlich macht.

Es können zusätzlich zu den dargestellten Elementen noch Abstandshalter zwischen den Ringen 6, 7 vorgesehen sein, die nicht formveränderlich sein müssen und ihre Gestalt beim Übergang zwischen der komprimierten und der expandierten Form des Rotors behalten können. Diese können als parallel zur Nabe 2 verlaufende Stangen bzw. Streben ausgebildet sein.

Grundsätzlich können die Ringe 6, 7 auch aus einem elastischen Material, beispielsweise gummiartigen Material bestehen, das beim Transport zum Einsatzort in komprimierter Form geringe Rückstellkräfte aufweist und sich nach Einnahme der Kreisringform selbst stabilisiert.

Die Fig. 11 zeigt eine axiale Draufsicht auf den Rotor gemäß Fig. 7 und die Fig. 12 einen Schnitt, bei dem das Schaufelblatt 1 die Zeichenebene am oberen und unteren Ende des Rotors schneidet. Das Schaufelblatt durchläuft zwischen dem oberen und dem unteren Ende des Rotors 180 Grad einer Wendel.

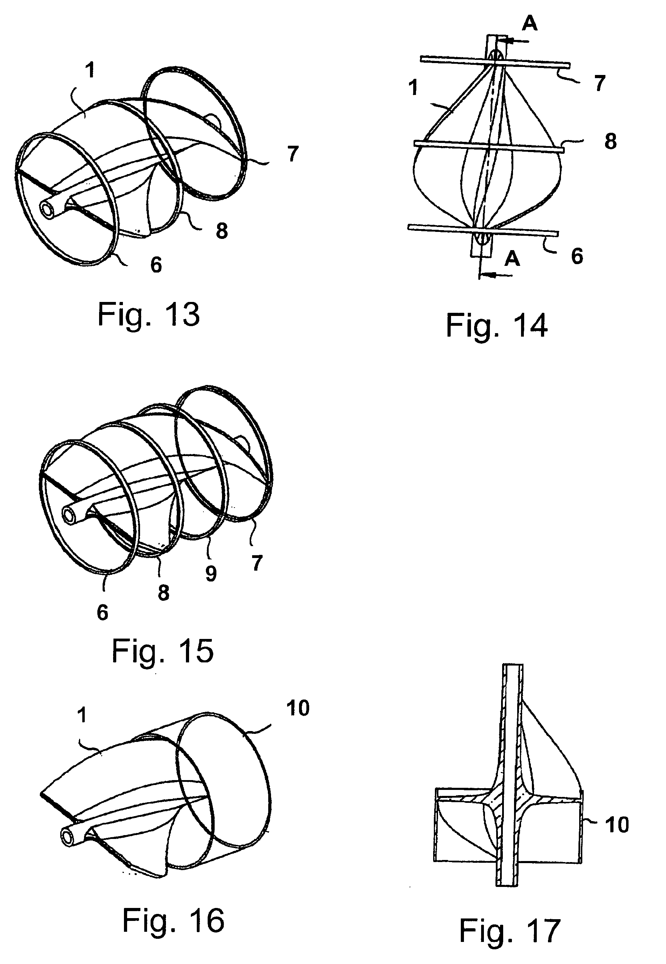

In der Fig. 13 ist ein Rotor einer Fluidpumpe dargestellt, der eine Stützeinrichtung mit drei Ringen 6, 7, 8 aufweist, die jeweils radial gesehen im Randbereich des Schaufelblattes 1 mit diesem verbunden sind und zueinander mittels nicht dargestellter Streben beabstandet sein können.

Fig. 14 zeigt eine Seitenansicht des Rotors aus Fig. 13.

In der Fig. 15 ist ein Rotor mit vier Ringen 6, 7, 8, 9 perspektivisch dargestellt, die zusammen den wesentlichen Teil
einer Stützeinrichtung bilden. Im Übrigen gilt für die Ringe 6, 7, 8, 9 das zu den oben beschriebenen Ausführungsformen bereits Ausgeführte.

Grundsätzlich kann das Drehmoment in den Rotor über die Stützeinrichtung eingebracht werden. Dazu muss ein Teil der Stützeinrichtung beispielsweise über Streben mit einer Antriebseinrichtung bzw. einer Antriebswelle verbunden sein. Auf diese Ankopplung wird weiter unten noch genauer eingegangen.

Alternativ kann das Drehmoment auch über die Nabe 2 eingebracht werden, sofern eine solche vorhanden ist. In diesem Fall beschränkt sich die Wirkung der Stützeinrichtung auf die radiale Stützung und Formgebung des Schaufelblattes / der Schaufelblätter.

Fig. 16 zeigt eine Ausführungsform in dreidimensionaler Darstellung, bei der ein Schaufelblatt 1 an seinem Umfang durch einen Rohrabschnitt 10 bzw. einen Schlauchabschnitt stabilisiert ist. Fig. 17 zeigt dieselbe Anordnung in einem Längsschnitt.

Der Rohrabschnitt 10 kann beispielsweise aus einem Kunststoff bestehen und einstückig mit dem Schaufelblatt hergestellt sein, jedoch auch aus einem vom Material des Schaufelblattes unterschiedlichen Material, beispielsweise einer Formgedächtnislegierung. Ein derartiger Schlauch- oder Rohrabschnitt hat gegenüber einem Ring den Nachteil, möglicherweise schwieriger komprimierbar zu sein, jedoch den Vorteil, in sich in expandierter Form leichter stabilisierbar zu sein.

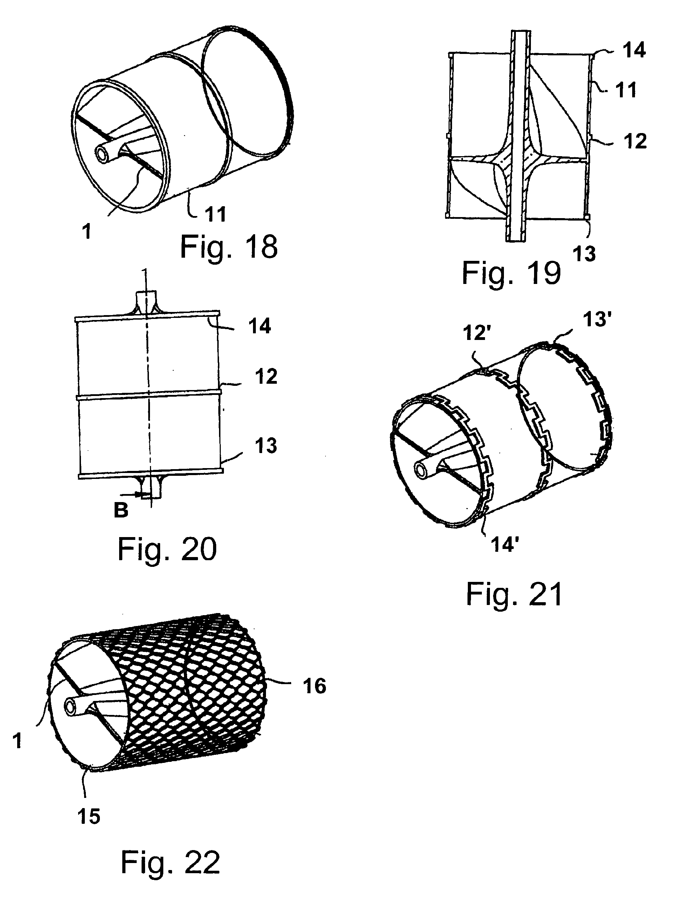

Fig. 18 zeigt in dreidimensionaler Ansicht einen Rotor einer Fluidpumpe mit einem Rotorblatt 1, das durch eine Stützeinrichtung 11 in Form eines durchgehenden Schlauchelementes gestützt ist. Das Schlauchelement 11 erstreckt sich über die volle axiale Länge des Schaufelblattes. Das Schlauchelement kann jedoch auch axial kürzer sein als das Schaufelblatt 1.

Fig. 19 zeigt einen Längsschnitt durch die Ausgestaltung aus Fig. 18, wobei insbesondere drei ringartige Verstärkungselemente 12, 13, 14 erkennbar sind, die radial außen an dem Schlauchelement 11 befestigt oder mit diesem einstückig zusammenhängend hergestellt sind.

Die Verstärkungselemente 12, 13, 14 können aus demselben Material bestehen wie das Schlauchelement 11, jedoch auch aus einem anderen, tendenziell steiferen Material, beispielsweise aus einer Formgedächtnislegierung aus einem Gummi, das tendenziell steifer sein kann als das flexible Material, aus dem das Schlauchelement 11 besteht.

Insofern kann bei der Entfaltung das Schlauchelement 11 durch Expansion der ringförmigen Verstärkungselemente 12, 13, 14 zumindest teilweise mitexpandiert werden. Diese Expansionsbewegung kann zudem durch einen in dem Rotor aufgebauten Überdruck verstärkt werden, sobald der Rotor in Rotation versetzt wird.

Fig. 20 zeigt eine Seitenansicht des Rotors aus Fig. 18, 19 in geschlossener Form.

In der Fig. 21 ist eine Variante dargestellt, bei der die ringförmigen Verstärkungselemente 12', 13', 14' in Umfangsrichtung mäanderförmig ausgebildet sind. Diese Verstärkungselemente können aus einem Formgedächtnismaterial bestehen und sind besonders durch die mäanderförmige Gestaltung leicht zusammenfaltbar. Außer der dargestellten, rechteckig mäanderförmigen Struktur können diese Verstärkungselemente auch eine Sägezahnstruktur oder eine Wellenlinienstruktur aufweisen.

Derartige Verstärkungselemente können beispielsweise auf das Schlauchelement aufgeklebt sein.

Fig. 22 zeigt in dreidimensionaler Darstellung ein Schlauchelement 15, das eine Stützeinrichtung für das Schaufelrad 1 bildet und auf seiner Außenseite durch ein Maschendrahtgeflecht 16 verstärkt ist. Das Maschendrahtgeflecht 16 kann beispielsweise auf das Schlauchelement 15 aufgeklebt sein, insbesondere auch nur punktuell. Das Maschendrahtgeflecht kann, wie bei Stents üblich, so ausgebildet sein, dass eine radiale Komprimierung nicht zu einer Änderung der Länge des Maschendrahtelementes führt. Das Maschendrahtgeflecht kann als Metalldraht oder auch als, insbesondere einstückige, Gitterstruktur aus einer Formgedächtnislegierung bestehen, jedoch ist auch eine Herstellung aus einem Kunststoff denkbar.

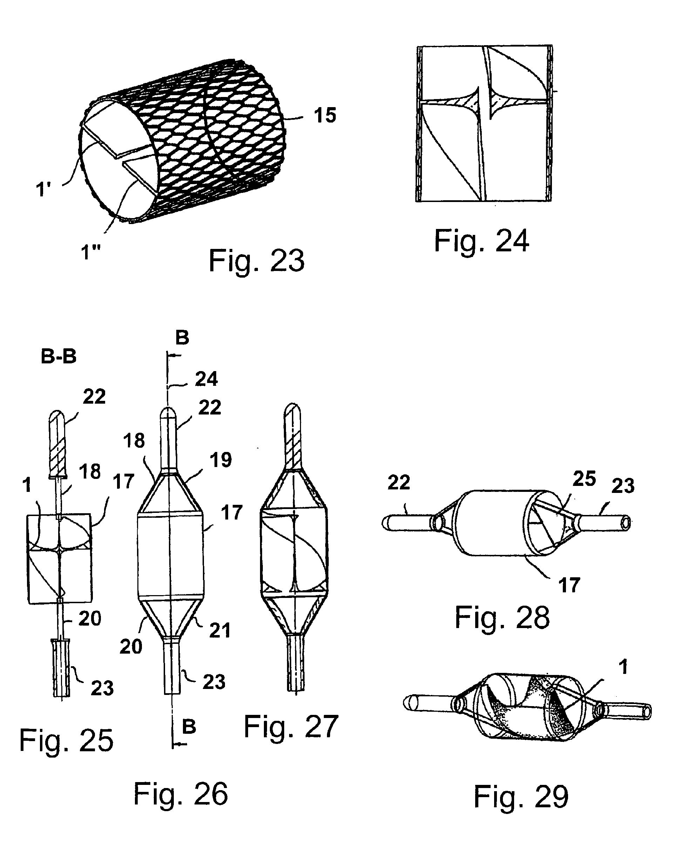

Die Fig. 23 zeigt eine Ausgestaltung eines Rotors mit zwei Schaufelblättern 1', 1", die jeweils an ihrer Außenseite wendelförmig in einem schlauchförmigen Element 15 befestigt, beispielsweise angeklebt sind, wobei die beiden Wendelformen derart aufeinander abgestimmt sind, dass die beiden Schaufelblätter 1', 1" bei Kompression der Stützeinrichtung 15 aneinander radial in bezug auf die Rotationsachse vorbeigleiten können, so dass eine weitgehende Kompression des Rotors insgesamt möglich ist.

Beide Schaufelblätter 1', 1" enden radial mit Abstand von der Rotationsachse, wobei eine Nabe nicht vorhanden ist. An dem rotationsachsennahen Rand der Schaufelblätter wirken nur geringe Kräfte auf diese durch das zu fördernde Fluid, da die Relativbewegung in diesem achsennahen Bereich gering ist. Der durch das Weglassen der Nabe eingesparte Raum kann zusätzlich zum Transport des Fluids genutzt werden, und das Fehlen der Nabe sowie die Zusammenschiebbarkeit der Schaufelblätter macht eine weitgehende Kompression des Rotors in radialer Richtung möglich.

Fig. 24 zeigt die Ausgestaltung aus Fig. 23 in einem Längsschnitt.

Ähnlich wie bei der Gestaltung aus Fig. 22 besteht die schlauchförmige Hülle, die die Stützeinrichtung für die Schaufelblätter bildet, aus einer inneren flexiblen Hülle und einem äußeren Maschengeflecht.

Die Fign. 25-29 zeigen insbesondere die Ankopplung der Stützeinrichtung an zwei Wellenzapfen die beiderseits des Rotors vorgesehen sind und sowohl einer Lagerung als auch dem Einleiten eines Drehmoments dienen können.

Fig. 25 zeigt in einer Längsschnittdarstellung eine schlauchförmige Stützeinrichtung 17, die über Streben 18,19,20,21 mit zwei Wellenzapfen 22, 23 verbunden ist. Da das Schaufelblatt 1 durch die Stützeinrichtung 17 getragen ist, wird der gesamte Rotor über die Wellenzapfen 22, 23 drehbar gelagert und angetrieben.

Fig. 26 zeigt eine um die Rotationsachse 24 um 90° gedrehte Darstellung in einer Seitenansicht.

Fig. 27 zeigt eine Ansicht aus derselben Richtung wie Fig. 26 in einer Schnittdarstellung.

Die Fign. 28, 29 zeigen von dem Gegenstand der Fign. 25, 26, 27 jeweils eine dreidimensionale Darstellung, wobei die Fig. 28 eine Außenansicht zeigt, während Fig. 29 eine teilweise aufgebrochene Darstellung zeigt, in der innerhalb der Stützeinrichtung 17 das Schaufelblatt 1 sichtbar wird.

Die Fign. 28, 29 zeigen im Unterschied zu den Fign. 25, 26, 27 als Verbindung zwischen der Stützeinrichtung 17 und den Wellenzapfen 22, 23 nicht gabelförmige Streben 18, 19, sondern eine dreieckige Platte 25. Diese hat gegenüber zwei gabelförmigen Streben den Vorteil, stabiler zu sein, jedoch den Nachteil, dass bei einer Drehbewegung mit hoher Rotationsfrequenz das Fluid durch die Platte 25 verdrängt werden muss.

Aus der Fig. 29 wird sichtbar, dass ein einziges wendelförmiges Schaufelblatt 1 vorgesehen ist, das keine Nabe aufweist.

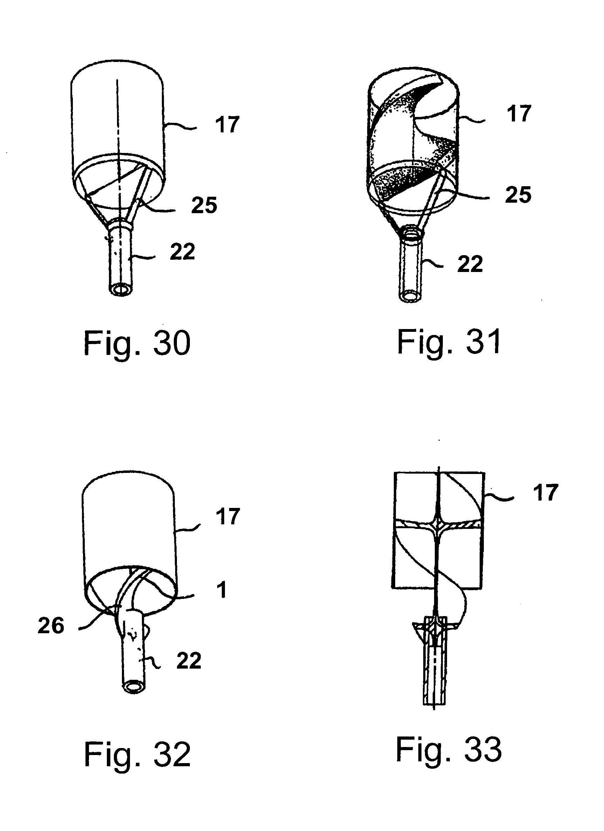

Die Fig. 30 zeigt eine Stützeinrichtung, bei der von dem schlauchartigen Element 17 aus in den Wellenzapfen 22 mittels einer dreieckigen Platte 25 das Drehmoment übertragen wird. Fig. 31 zeigt dieselbe Konstellation in einer teilweise durchbrochenen Darstellung.

Im Gegensatz dazu zeigt Fig. 32 die Verbindung des Wellenzapfens 22 direkt mit einem Fortsatz 26 des Schaufelblattes 1.

Fig. 33 zeigt dieselbe Konstellation wie Fig. 32 in einem Längsschnitt. Die Fign. 32 und 33 machen deutlich, dass dort das Drehmoment direkt in das Schaufelblatt 1 eingebracht wird und dass die Stützeinrichtung 17 lediglich zur Stabilisierung des Schaufelblattes / der Schaufelblätter radial im Außenbereich bzw. zur Expansion des Schaufelblattes und zur nachfolgenden Formstabilisierung dient. Die Fortsetzung der wendelförmigen Struktur 26 bis zum Wellenzapfen 22 hat zudem den Vorteil, dass dies auch bei
Rotation wenig Widerstand in dem umgebenden Fluid hervorruft, da es eine Fortsetzung der wendelförmigen Struktur des Schaufelblattes darstellt.

Anhand der oben beschriebenen Beispiele wurde verdeutlicht, dass mit den Mitteln der Erfindung durch die Stützung von einem oder mehreren Schaufelblättern radial in deren Außenbereich mit geringem Aufwand eine hohe Stabilisierung der Schaufelblätter erreicht wird. Die Verbindung von Schaufelblättern mit einer Nabe wird dadurch entweder nicht besonders beansprucht oder sie wird insgesamt unnötig, was auch zum Entfallen der Nabe führen kann. Die Stützeinrichtung kann zudem bei der Expansion des Rotors das Schaufelblatt / die Schaufelblätter mitexpandieren, so dass diese aus handelsüblichen flexiblen Materialien ohne besonders hohe mechanische Anforderungen hergestellt werden können.

## Patentansprüche

1. Fluidpumpe mit wenigstens einem Schaufelblatt (1,1',1"), das um eine Rotationsachse (3) drehbar ist und im Betrieb ein Fluid fördert und mit einer Stützeinrichtung
(4,6,7,8,9,10,12,12',13,13',14,14',15,17), die das wenigstens eine Schaufelblatt in wenigstens einem Stützbereich stützt, wobei die Stützeinrichtung zwischen einem ersten Zustand, in dem der Rotor radial komprimiert ist, und einem zweiten Zustand, in dem der Rotor radial expandiert ist, veränderbar ist und wobei sich wenigstens ein Schaufelblatt im radial expandierten Zustand des Rotors wenigstens teilweise in Bezug auf die Rotationsachse (3) von dem Stützbereich / den Stützbereichen aus radial nach innen erstreckt.

2. Fluidpumpe nach Anspruch 1, **dadurch gekennzeichnet, dass** sich der überwiegende Teil des Fluid fördernden Bereichs des Schaufelblattes / der Schaufelblätter (1,1',1") radial innerhalb des Stützbereiches / der Stützbereiche erstreckt.

3. Fluidpumpe nach Anspruch 2, **dadurch gekennzeichnet, dass** der Stützbereich radial außen am Umfang des Fluid fördernden Bereiches der Schaufelblätter (1,1',1") angeordnet ist.

4. Fluidpumpe nach Anspruch 1, 2 oder 3, **dadurch gekennzeichnet, dass** das Schaufelblatt / die Schaufelblätter (1,1',1") mit der Stützeinrichtung (4,6,7,8,9,10,12,12',13,13',14,14',15,17) verbunden ist und diese mit dem Schaufelblatt / den Schaufelblättern rotiert.

5. Fluidpumpe nach Anspruch 1, 2 oder 3, **dadurch gekennzeichnet, dass** das wenigstens eine Schaufelblatt (1,1',1") beweglich gegenüber der Stützeinrichtung
(4,6,7,8,9,10,12,12',13,13',14,14',15,17) geführt und gelagert ist.

6. Fluidpumpe nach einem der Ansprüche 1 bis 5, **dadurch gekennzeichnet, dass** die Stützeinrichtung durch wenigstens einen zur Rotationsachse konzentrisch gelagerten Ring
(6,7,8,9,12,12',13,13',14,14',) gebildet ist.

7. Fluidpumpe nach Anspruch 6, **dadurch gekennzeichnet, dass** der wenigstens eine Ring (12',13',14') im Umfangsrichtung mäanderförmig gestaltet ist.

8. Fluidpumpe nach Anspruch 6 oder 7, **dadurch gekennzeichnet, dass** die Stützeinrichtung zwei oder mehr axial voneinander beabstandete Ringe (6,7,8,9) aufweist.

9. Fluidpumpe nach einem der Ansprüche 1 bis 5, **dadurch gekennzeichnet, dass** die Stützeinrichtung durch einen das Schaufelblatt / die Schaufelblätter wenigstens teilweise umgebenden, flexiblen Schlauch (10,15,17) gebildet ist.

10. Fluidpumpe nach Anspruch 9, **dadurch gekennzeichnet, dass** der Schlauch(10,15,17) im Pumpbetrieb durch den Überdruck infolge des Pumpbetriebs aufblasbar und formstabil ist.

11. Fluidpumpe nach Anspruch 1 oder einem der folgenden, **dadurch gekennzeichnet, dass** das Schaufelblatt/die Schaufelblätter radial mit allen seinen Teilen von der Rotationsachse (3) beabstandet ist/sind.

12. Fluidpumpe nach Anspruch 1 oder einem der folgenden, **dadurch gekennzeichnet, dass** die Fluidpumpe im Bereich des Schaufelblatts / der Schaufelblätter keine Nabe aufweist.

13. Fluidpumpe nach Anspruch 1 oder einem der folgenden, **dadurch gekennzeichnet, dass** der Ring / die Ringe (6,7,8,9) aus einem elastischen Material, insbesondere einem Formgedächtnismaterial, besteht / bestehen.

14. Fluidpumpe nach Anspruch 1 oder einem der folgenden, **dadurch gekennzeichnet, dass** die Stützeinrichtung einen im Durchmesser komprimierbaren Maschendrahtzylinder (16) aufweist.

15. Fluidpumpe nach Anspruch 1 oder einem der folgenden, **dadurch gekennzeichnet, dass** die Stützeinrichtung
(4,6,7,8,9,10.12.12',13,13',14,14',15,17) in wenigstens einem Rotationslager gelagert ist, das / die axial außerhalb des Bereiches angeordnet ist / sind, in dem sich das Schaufelblatt / die Schaufelblätter erstreckt / erstrecken.
